# EUROPEAN PATENT APPLICATION

(11) **EP 4 524 564 A1**
(43) Date of publication of application: **19.03.2025**
(21) Application number: 22941570.8
(22) Date of filing: 31.12.2022
(51) Int. Cl.: G01N 33/52

(54) **TESTING APPARATUS FOR TESTING COAGULATION ITEM AND REAGENT CARD**

(30) Priority: 12.05.2022 CN 202210520886
(71) Applicant: Shenzhen Dymind Biotechnology Co., Ltd, Shenzhen, Guangdong 518107 (CN)
(72) Inventor: XIAO, Hua, Shenzhen, Guangdong 518107 (CN); LIANG, Guolv, Shenzhen, Guangdong 518107 (CN); ZHANG, Zhiguo, Shenzhen, Guangdong 518107 (CN); HU, Yanyong, Shenzhen, Guangdong 518107 (CN)
(74) Representative: Herrero & Asociados, S.L.
(86) International application number: PCT/CN2022/144438
(87) International publication number: WO 2023/216638

(57) **Abstract**

The present application discloses a testing apparatus for testing a coagulation item and a reagent card. The testing apparatus comprises: a reagent storage module that comprises a multi-person reagent storage module and a single-person reagent storage module, the multi-person reagent storage module being used for storing reagent bottles, the single-person reagent storage module comprising at least one reagent card storage channel to store a reagent card, and the reagent card comprising at least one reagent storage position to store a single-person test reagent; a distribution module that is used for injecting a collected sample into a test cup and injecting a test reagent in a reagent bottle or a single-person test reagent in a reagent card into the test cup; and an incubation test module that is used for incubating the sample in the test cup and testing the sample in the test cup to obtain a coagulation test result. **In** this way, the requirements of the testing apparatus for single-person sample testing can be met, and the waste of large bottles of reagents is also avoided.

## Description

### CROSS REFERENCE TO RELATED APPLICATIONS

The present application claims priority to Chinese Patent Application No. 202210520886.7, filed May 12, 2022, entitled "DETECTION DEVICE FOR DETECTING COAGULATION ITEM AND REAGENT KIT FOR DETECTING COAGULATION ITEM", which is herein incorporated by reference in its entirety.

### TECHNICAL FIELD

The present disclosure relates to the field of medical devices, and in particular to a detection device for detecting a coagulation item, and a reagent kit for detecting a coagulation item.

### BACKGROUND

A detection device for detecting a coagulation item in the related art is configured to detect a coagulation ability of a sample, and includes a sample storage module and a reagent storage module. The sample storage module is configured to store a plurality of test tubes. The reagent storage module is configured to store a plurality of reagent bottles. The plurality of reagent bottles are configured to hold a large volume of reagents, which may meet the requirement of dozens of detection samples and may be suitable for a scenario with a large volume of samples. However, in a case of a small volume of samples (for example, there is only several detection samples), since the reagent cannot be used up, the reagent bottle with a limited shelf life may be wasted after the reagent bottle is opened. Therefore, the detection device in the related art is not suitable for detecting a single sample.

A technical problem may be that the detection device in the related art is not suitable for detecting the single sample.

### SUMMARY OF THE DISCLOSURE

In order to solve the above technical problems, the embodiments of the present disclosure provide a detection device for detecting a coagulation item. The detection device for detecting a coagulation item includes: a reagent storage module, including a single-serving reagent storage module, where the single-serving reagent storage module includes at least one reagent kit storage channel to store a reagent kit, and the reagent kit includes at least one reagent storage unit to store a single-serving detection reagent, so as to perform a single-serving coagulation detection; a dispensing module, configured to inject a collected sample into a detection cup, and inject the single-serving detection reagent in the reagent kit into the detection cup; and an incubation and detection module, configured to incubate the sample in the detection cup, and detect the sample in the detection cup to obtain a coagulation detection result.

In some embodiments, the detection device further includes a sample storage module, configured to store a test tube, where the dispensing module is configured to inject a sample in the test tube into the detection cup; and a scanning module, configured to scan identification information of the test tube, so as to enable the detection device to bind the identification information with the coagulation detection result.

In some embodiments, the detection device further includes a detection cup storage module configured to store the detection cup, where the dispensing module is configured to inject the collected sample into the detection cup in the detection cup storage module, and inject the single-serving detection reagent in the reagent kit into the detection cup in the detection cup storage module.

In some embodiments, the detection device further includes a grabbing module configured to move the detection cup from the detection cup storage module to the incubation and detection module.

In some embodiments, the reagent storage module includes a multi-serving reagent storage module, the multi-serving reagent storage module is configured to store a reagent bottle to perform a multi-serving coagulation detection, and the dispensing module is configured to inject the detection reagent in the reagent bottle or the single-serving detection reagent in the reagent kit into the detection cup.

In some embodiments, the reagent kit includes a first reagent kit, and the first reagent kit includes at least one reagent storage unit configured to store the single-serving detection reagent; and the at least one reagent kit storage channel includes a first reagent kit storage channel configured to place the first reagent kit.

In some embodiments, the reagent kit includes a second reagent kit, the second reagent kit includes at least one reagent storage unit and at least one detection unit, the at least one reagent storage unit is configured to store the single-serving detection reagent, and the at least one detection unit is configured to serve as the detection cup; the dispensing module is configured to inject the sample into the detection unit, and inject the detection reagent in the reagent bottle or the single-serving detection reagent in the reagent kit into the detection unit; and the at least one reagent kit storage channel includes a second reagent kit storage channel configured to place the second reagent kit.

In some embodiments, the reagent kit includes a third reagent kit, the third reagent kit includes at least one reagent storage unit and a sample storage unit, the at least one reagent storage unit is configured to store the single-serving detection reagent, and the sample storage unit is configured to store the sample; and the at least one reagent kit storage channel includes a third reagent kit storage channel configured to place the third reagent kit.

In some embodiments, the reagent kit includes a fourth reagent kit, the fourth reagent kit includes at least one reagent storage unit, at least one detection unit, and a sample storage unit, the reagent storage unit is configured to store the single-serving detection reagent, the sample storage unit is configured to store the sample, and the detection unit is configured to serve as the detection cup; the dispensing module is configured to inject the sample into the detection unit, and inject the detection reagent in the reagent bottle or the single-serving detection reagent in the reagent kit into the detection unit; and the at least one reagent kit storage channel includes a fourth reagent kit storage channel configured to place the fourth reagent kit.

In some embodiments, the incubation and detection module includes a first incubation detection channel; and in response to the first reagent kit being stored in the first reagent kit storage channel or the third reagent kit being stored in the third reagent kit storage channel to perform the single-serving coagulation detection, or in response to the detection device performing multi-serving coagulation detection, the dispensing module is configured to inject the sample and the single-serving detection reagent into the detection cup, respectively, or the dispensing module is configured to inject the sample and a detection reagent in the reagent bottle into the detection cup, respectively, and the detection cup is transferred to the first incubation detection channel.

In some embodiments, the incubation and detection module includes a second incubation detection channel arranged correspondingly to the second reagent kit storage channel and/or the fourth reagent kit storage channel, in response to the second reagent kit being placed into the second reagent kit storage channel or the fourth reagent kit being placed into the fourth reagent kit storage channel, the at least one detection unit in the second reagent kit or the at least one detection unit in the fourth reagent kit is placed into the second incubation detection channel, such that the at least one detection unit in the second reagent kit or the at least one detection unit in the fourth reagent kit is configured to serve as the detection cup, and the dispensing module is configured to inject the sample and the single-serving detection reagent into the at least one detection unit in the second reagent kit or the at least one detection unit in the fourth reagent kit, or the dispensing module is configured to inject the sample and the detection reagent in the reagent bottle into the at least one detection unit in the second reagent kit or the at least one detection unit in the fourth reagent kit.

In some embodiments, the first incubation detection channel is an optical detection channel.

In some embodiments, the optical detection channel includes an optical detection module. the optical detection module includes: a light source generator, configured to emit a light beam, where the light beam irradiates to the sample to be detected in the detection cup to generate scattered light or transmitted light; and a photodetector, configured to detect the scattered light or the transmitted light, and configured to convert an optical signal of the scattered light or an optical signal of the transmitted light into an electrical signal to obtain the detection result.

In some embodiments, the first incubation detection channel is a magnetic bead detection channel.

In some embodiments, the magnetic bead detection channel includes a magnetic bead detection module, and a groove is defined on the detection cup, so as to cause a magnetic bead to roll in the groove. The magnetic bead detection module includes: an alternating coil, configured to drive the magnetic bead to roll; and a detection coil, arranged in a plane perpendicular to the alternating coil, and configured to detect an induced current which is generated by cutting the alternating coil during a rolling process of the magnetic bead to obtain the detection result.

In some embodiments, the first incubation detection channel is a combined detection channel for optical detection and magnetic bead detection.

In some embodiments, the combined detection channel for optical detection and magnetic bead detection includes: a light source generator, a photodetector, an alternating coil, and a detection coil, the light source generator and the photodetector are arranged opposite to each other and are disposed above the alternating coil and the detection coil, and the detection coil is arranged in a plane perpendicular to the alternating coil.

In order to solve the above technical problems, the embodiments of the present disclosure provide a reagent kit. The reagent kit includes a main body and the at least one reagent storage unit, and the at least one reagent storage unit is arranged on the main body, where the at least one reagent storage unit is configured to store the single-serving detection reagent, such that the detection device is configured to perform the single-serving coagulation detection.

In some embodiments, the reagent kit further includes a handle arranged at an end of the main body.

In some embodiments, the reagent kit includes a sample storage unit configured to place the sample.

In some embodiments, the reagent kit includes at least one detection unit configured to serve as the detection cup of the detection device.

Different from the related art, the detection device for detecting the coagulation item is provided by the embodiments of the present disclosure. The detection device includes: the reagent storage module, the dispensing module, and the incubation and detection module. The reagent storage module includes the multi-serving reagent storage module and the single-serving reagent storage module. The multi-serving reagent storage module is configured to store the reagent bottle, so as to perform the multi-serving coagulation detection. The single-serving reagent storage module includes the at least one reagent kit storage channel, and the at least one reagent kit storage channel is configured to store the reagent kit. The reagent kit includes the at least one reagent storage unit, and the at least one reagent storage unit is configured to store the single-serving detection reagent, so as to perform the single-serving coagulation detection. The dispensing module is configured to inject a collected sample into a detection cup, and inject the single-serving detection reagent in the reagent kit into the detection cup. The incubation and detection module is configured to incubate the sample in the detection cup, and detect the sample in the detection cup to obtain a coagulation detection result. Therefore, the detection device may store the reagent kit through the single-serving reagent storage module, and the at least one reagent storage unit of the reagent kit is configured to store the single-serving detection reagent, such that the single-serving detection reagent may be supplied to the detection device without using the reagent bottle in the multi-serving reagent storage module, thereby enabling the detection device to achieve a single sample detection, and thus it may be further possible to reduce waste of a large bottle of reagent. A detection method of the detection device is diverse, such that it may be possible to meet detection requirements of a user in different situations, thereby improving the user's experience and the convenience of detection.

### BRIEF DESCRIPTION OF THE DRAWINGS

In order to illustrate the technical solutions more clearly in the embodiments of the present disclosure, the following will be briefly described in the description of the embodiments required to use the accompanying drawings. It is obvious that the accompanying drawings are only some of the embodiments of the present disclosure, and those skilled in the art, without creative work, can also obtain other accompanying drawings based on these drawings.
FIG. 1 is a structural schematic view of a first embodiment of a coagulation detection device according to the present disclosure.
FIG. 2 is a structural schematic view of a first embodiment of a reagent kit according to the present disclosure.
FIG. 3 is a structural schematic view of a first embodiment of an optical detection module according to the present disclosure.
FIG. 4 is a structural schematic view of a first embodiment of a magnetic bead detection module according to the present disclosure.
FIG. 5 is a structural schematic view of a first embodiment of the optical detection module and the magnetic bead detection module according to the present disclosure.
FIG. 6 is a structural schematic view of a second embodiment of the reagent kit according to the present disclosure.
FIG. 7 is a structural schematic view of a third embodiment of the reagent kit according to the present disclosure.
FIG. 8 is a structural schematic view of a fourth embodiment of the reagent kit according to the present disclosure.

Reference numerals in drawings: reagent storage module 11; multi-serving reagent storage module 111; single-serving reagent storage module 112; reagent bottle 113; common reagent bottle 114; reagent kit storage channel 115; reagent kit 116; reagent storage unit 117; handle 118; sample storage unit 119; detection unit 120; dispensing module 12; incubation and detection module 13; first incubation detection channel 131; second incubation detection channel 132; detection cup 14; sample storage module 15; test tube 151; scanning module 16; detection cup storage module 17; grabbing module 18; display screen 19; barcode scanner 20; cleaning solution module 21; cleaning solution barrel 211; first pump body 212; waste liquid module 22; waste liquid barrel 221; second pump body 222; power source 23; cleaning pool 24; printer 25; waste cup barrel 26; optical detection module 30; light source generator 31; photodetector 32; magnetic bead detection module 40; alternating coil 41; detection coil 42; combined detection module for optical detection and magnetic bead detection 50; light source generator 51; photodetector 52; alternating coil 53; detection coil 54.

### DETAILED DESCRIPTION

In order to make the above purposes, features and advantages of the present disclosure more obvious and understandable, the specific implementation mode of the present disclosure is described in detail below in combination with the drawings. It is understood that the specific embodiments described herein are only used to explain the present disclosure, but not to limit the present disclosure. In addition, it should be noted that, for the convenience of description, only part of the structures related to the present disclosure are shown in the drawings, but not all the structures. Based on the embodiments in the present disclosure, all other embodiments obtained by those skilled in the art without doing creative work belong to the protection scope of the present disclosure.

Terms "first", "second", etc., in the present disclosure are used to distinguish different objects, and are not intended to describe a specific order. Furthermore, terms "comprise/include" and "arranged with" and variations thereof are intended to cover a non-exclusive inclusion. For example, a process, a method, a system, a product, or a device that includes a series of steps or units is not limited to the listed steps or units, but optionally includes steps or units not listed, or optionally include other steps or units inherent to these processes, methods, systems, products or devices.

"Embodiment" herein means that a particular feature, structure, or characteristic described with reference to embodiments may be included in at least one embodiment of the present disclosure. The term appearing in various places in the specification are not necessarily as shown in the same embodiment, and are not exclusive or alternative embodiments that are mutually exclusive with other embodiments. Those skilled in the art will understand explicitly and implicitly that the embodiments described herein may be combined with other embodiments.

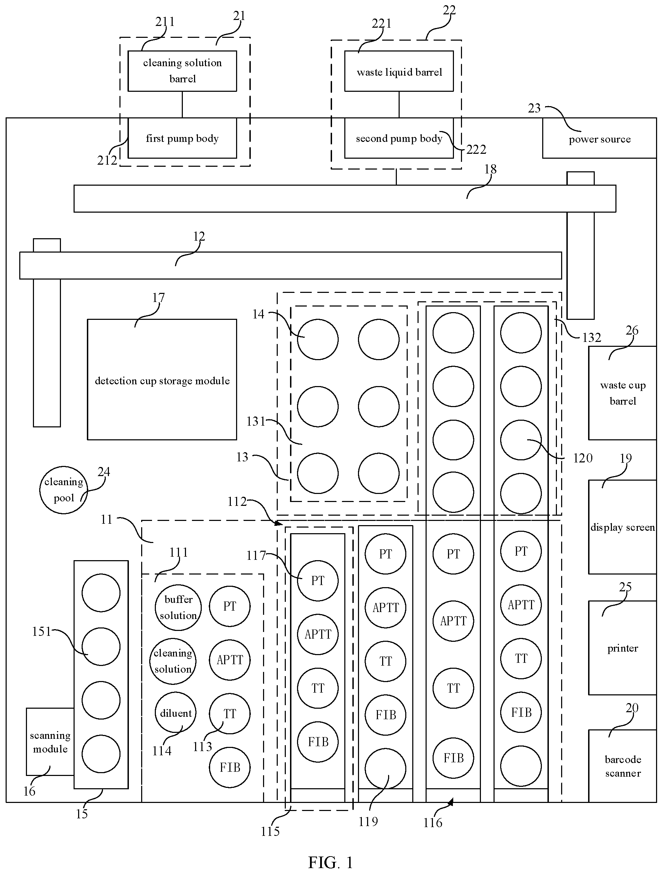

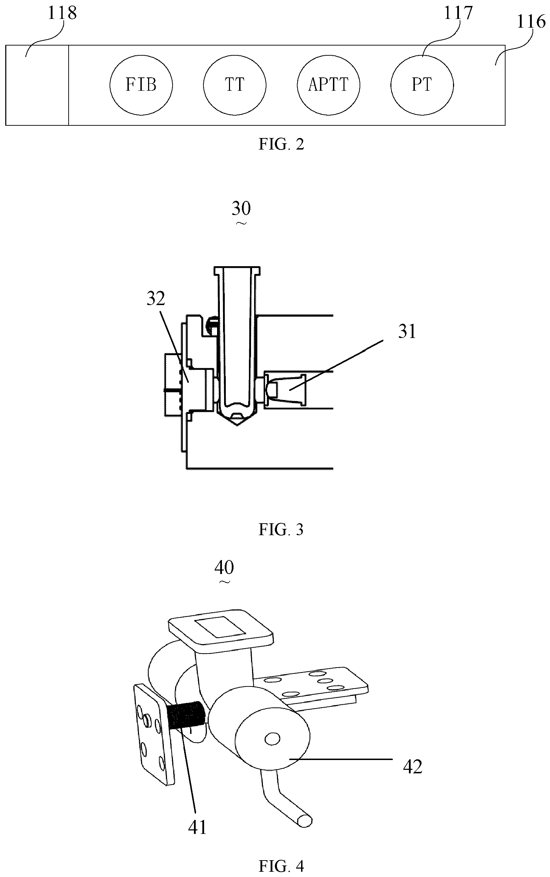

As shown in FIGS. 1-2, FIG. 1 is a structural schematic view of a first embodiment of a coagulation detection device according to the present disclosure, and FIG. 2 is a structural schematic view of a first embodiment of a reagent kit (i.e., a reagent assembly) according to the present disclosure. The coagulation detection device provided in the embodiments of the present disclosure is configured to detect at least one coagulation item. The coagulation detection device includes a reagent storage module 11, a dispensing/aspiration module 12, an incubation and detection module 13, and a detection cup/detection cuvette 14.

The reagent storage module 11 includes a multi-serving reagent storage module 111 and a single-serving reagent storage module 112. The multi-serving reagent storage module 111 is configured to store a reagent bottle 113, so as to perform a multi-serving coagulation detection. The reagent bottle 113 stored in the multi-serving reagent storage module 111 is configured to store a detection reagent, and the detection reagent includes at least one of: a prothrombin time (PT) reagent, an activated partial thromboplastin time (APTT) reagent, a thrombin time (TT) reagent, and a fibrinogen (FIB) reagent.

In some embodiments, the multi-serving reagent storage module 111 is further configured to store a common reagent bottle 114, and the common reagent bottle 114 is configured to store a buffer solution, a cleaning solution and/or a diluent. The common reagent bottle 114 and the reagent bottle 113 may be arranged in an array.

The single-serving reagent storage module 112 includes at least one reagent kit storage channel 115. The at least one reagent kit storage channel 115 is configured to store a reagent kit 116 (i.e., a reagent assembly 116). The reagent kit 116 includes at least one reagent storage unit/reagent storage unit 117. The at least one reagent storage unit 117 is configured to store a single-serving detection reagent, so as to perform a single-serving coagulation detection. In some embodiments, the reagent kit 116 includes four reagent storage units 117, and four reagents, which are stored in the four reagent storage units 117 respectively, are a PT reagent, an APTT reagent, a TT reagent, and a FIB reagent.

The dispensing module 12 is configured to inject a collected sample into the detection cup 14, and inject a detection reagent in the reagent bottle 113 or the single-serving detection reagent in the reagent kit 116 into the detection cup 14. In some embodiments, the sample in the dispensing module 12 is injected into the detection cup 14, and then the detection reagent in the reagent bottle 113 or the single-serving detection reagent in the reagent kit 116 is injected into the corresponding detection cup 14.

The incubation and detection module 13 is configured to incubate the sample in the detection cup 14, and detect the sample in the detection cup 14, so as to obtain a coagulation detection result.

When the single-serving coagulation detection is performed on the sample by the detection device, the dispensing module 12 is configured to inject the sample and the single-serving detection reagent into the detection cup 14, respectively, and the detection cup 14 is moved to the incubation and detection module 13 to incubate the sample and the single-serving detection reagent in the detection cup 14. The incubation and detection module 13 is configured to detect an incubated sample, and the coagulation detection result is obtained. When the multi-serving coagulation detection is performed on the sample by the detection device, the dispensing module 12 is configured to inject the sample and the detection reagent in the reagent bottle 113 into the detection cup 14, respectively, and the detection cup 14 is moved to the incubation and detection module 13 to incubate the sample and the detection reagent in the detection cup 14. The incubation and detection module 13 is configured to detect an incubated sample, and the coagulation detection result is obtained.

In some embodiments, the detection device is configured to perform four coagulation detection items (i.e., four coagulation indexes) on the sample. In some embodiments, the detection device is configured to perform a PT detection, an APTT detection, a TT detection, and a FIB detection on the sample.

In the embodiments, the reagent storage module 11 includes the multi-serving reagent storage module 111 and the single-serving reagent storage module 112. The multi-serving reagent storage module 111 is configured to store the reagent bottle 113, so as to perform the multi-serving coagulation detection. The single-serving reagent storage module 112 includes the at least one reagent kit storage channel 115, and the at least one reagent kit storage channel 115 is configured to store the reagent kit 116. The reagent kit 116 includes the at least one reagent storage unit 117, and the at least one reagent storage unit 117 is configured to store the single-serving detection reagent, so as to perform the single-serving coagulation detection. Therefore, the detection device may store the reagent kit 116 through the single-serving reagent storage module 112, and the at least one reagent storage unit 117 of the reagent kit 116 is configured to store the single-serving detection reagent, such that the single-serving detection reagent may be supplied to the detection device without using the reagent bottle 113 in the multi-serving reagent storage module 111, thereby enabling the detection device to achieve a single sample detection, and thus it may be further possible to reduce waste of a large bottle of reagent (such as the reagent in the reagent bottle 113). The detection device may improve the diversity of a detection method thereof, such that it may be possible to meet detection requirements of a user in different situations, thereby improving the user experience and the convenience of detection.

In some embodiments, the detection device includes a sample storage module 15, a scanning module 16, a detection cup storage module 17, and a grabbing module 18.

The sample storage module 15 is configured to store a test tube 151, and the test tube 151 may be configured to store the sample. The sample may be a whole blood sample. The dispensing module 12 may be configured to inject the sample in the test tube 151 into the detection cup 14.

The scanning module 16 is configured to scan identification information of the test tube 151. In some embodiments, the scanning module 16 is configured to scan a label or a barcode of the test tube 151 to obtain identification information. The detection device is configured to bind the identification information with the coagulation detection result, such that the test tube 151 may be bound with the coagulation detection result.

The detection cup storage module 17 is configured to store the detection cup 14. The detection cup storage module 17 is spaced apart from the incubation and detection module 13. The dispensing module 12 is configured to inject the collected sample into the detection cup 14 in the detection cup storage module 17, and inject the detection reagent in the reagent bottle 113 or the single-serving detection reagent in the reagent kit 116 into the detection cup 14 in the detection cup storage module 17.

The grabbing module 18 is configured to move the detection cup 14 from the detection cup storage module 17 to the incubation and detection module 13. In some embodiments, the grabbing module 18 includes a grabbing arm and a grabbing hand/grabhook. The grabbing arm is configured to grab the detection cup 14 through the grabbing hand, such that the detection cup 14 may be moved from the detection cup storage module 17 to the incubation and detection module 13.

In some embodiments, the reagent kit 116 includes a first reagent kit, and the first reagent kit includes at least one reagent storage unit 117. As shown in FIG. 2, the at least one reagent storage unit 117 is configured to store the single-serving detection reagent. The at least one reagent kit storage channel 115 of the single-serving dose reagent storage module 112 includes a first reagent kit storage channel. The first reagent kit storage channel is configured to store the first reagent kit.

In some embodiments, the reagent kit 116 includes a handle 118, and the handle 118 is arranged at an end of the reagent kit 116. The user can easily hold the reagent kit 116 through the handle 118. In addition, the reagent kit 116 further includes a reagent kit label, and the reagent kit label is arranged on the reagent kit 116 and is configured to indicate production information of the reagent kit 116.

In some embodiments, the single-serving detection reagent is a dry powder reagent/freeze-dried powder. Before the dispensing module 12 aspirates the single-serving detection reagent from the reagent storage unit 117, the dispensing module 12 adds a composite solution to the dry reagent in the reagent kit 116. In addition, a surface of the reagent kit 116 is sealed for storage and transportation.

In some embodiments, the incubation and detection module 13 includes a first incubation detection channel 131. When the first reagent kit is stored in the first reagent kit storage channel to perform the single-serving coagulation detection, or when the detection device performs the multi-serving coagulation detection, the dispensing module 12 injects the sample and the single-serving detection reagent into the detection cup 14, respectively, or the dispensing module 12 injects the sample and the detection reagent in the reagent bottle 113 into the detection cup 14, respectively. The detection cup 14 is transferred to the first incubation detection channel 131. In some embodiments, the grabbing module 18 may be configured to transfer the detection cup 14 to the first incubation detection channel 131.

A detection process of the detection device for performing the single-serving coagulation detection based on the first reagent kit may be described as follows. The grabbing module 18 moves the detection cup 14 from the detection cup storage module 17 to a liquid dispensing/aspiration position. The liquid dispensing position may be located in the detection cup storage module 17 or the incubation and detection module 13. The dispensing module 12 injects the sample in the test tube 151 of the sample storage module 15 into the detection cup 14, and the dispensing module 12 is cleaned. The dispensing module 12 aspirates the single-serving detection reagent from the reagent storage unit 117 of the first reagent kit, and injects the single-serving detection reagent into the detection cup 14. The grabbing module 18 moves the detection cup 14 from the liquid dispensing position to the first incubation detection channel 131 of the incubation and detection module 13. After the sample in the detection cup 14 and the single-serving detection reagent is incubated, the sample in the detection cup 14 may be detected through the first incubation detection channel 131, so as to calculate the coagulation detection result such as coagulation time of the sample, a concentration of the sample, an activity indicator of the sample, etc.

A detection process of the detection device for performing the multi-serving coagulation detection may be described as follows. The grabbing module 18 moves the detection cup 14 from the detection cup storage module 17 to the liquid dispensing position. The dispensing module 12 injects the sample in the test tube 151 of the sample storage module 15 into the detection cup 14, and the dispensing module 12 is cleaned. In some embodiments, the dispensing module 12 injects a certain amount of diluent or a certain amount of buffer solution in the common reagent bottle 114 into the detection cup 14, so as to dilute the sample in the detection cup 14. The dispensing module 12 aspirates the detection reagent from the reagent bottle 113 and injects the detection reagent into the detection cup 14. The grabbing module 18 moves the detection cup 14 from the liquid dispensing position to the first incubation detection channel 131 of the incubation and detection module 13. After the sample in the detection cup 14 and the single-serving detection reagent is incubated, the sample in the detection cup 14 may be detected through the first incubation detection channel 131, so as to calculate the coagulation detection result such as coagulation time of the sample, a concentration of the sample, an activity indicator of the sample, etc.

In some embodiments, after the detection device completes detection of the sample, the detection device performs cleaning and maintenance. Therefore, the detection device may not only meet requirements of large batch coagulation detection for multiple persons, but also meet requirements of the single-serving coagulation detection. In this way, it may be possible to achieve multiple uses of one machine, meet different needs of the user, improve the user's experience, and be easy to maintain.

In addition, the dispensing module 12 is configured to inject the collected sample into the detection cup 14, and inject the detection reagent in the reagent bottle 113 or the single-serving detection reagent in the reagent kit 116 into the detection cup 14, so as to perform a mixing operation. In some embodiments, a dispensing needle/probe of the dispensing module 12 may achieve the mixing by aspiration, dispensing, vibration, and other manners.

In some embodiments, the dispensing module 12 includes a plurality of dispensing needles/probes. The plurality of dispensing needles of the dispensing module 12 may simultaneously aspirate a plurality of samples from different test tubes 151 in the sample storage module 15, and inject the plurality of samples into a corresponding one of detection cups 14. In addition, the plurality of dispensing needles of the dispensing module 12 may simultaneously aspirate a single-serving detection reagent from a corresponding one of the plurality of reagent kits 116, and inject the single-serving detection reagents into the plurality of detection cups at the same time. The dispensing module 12 realizes simultaneous collection or simultaneous injection of the plurality of samples and simultaneous collection or simultaneous injection of the plurality of single-serving detection reagents, so as to improve a detection throughput of the detection device, thereby improving the detect efficiency.

In some embodiments, the detection device includes a reagent kit scheduling module. The reagent kit scheduling module is configured to move the reagent kit 116 to the reagent kit storage channel 115 when the detection device detects the sample, and move the reagent kit 116 out of the reagent kit storage channel 115 when the detection device completes the detection on the sample. Therefore, the reagent kit scheduling module may load and unload the reagent kit 116, so as to improve the automation of the detection device and improve the detect efficiency.

In some embodiments, the detection device includes a test tube scheduling module. The test tube scheduling module is configured to move a test tube rack to a preset position when the detection device detects the sample, and move the test tube rack out when the detection device completes the detection on the sample, so as to improve the automation of the detection device and simplify the operation.

In some embodiments, the detection device includes a telescopic mechanism, and the detection cup storage module 17 is mounted on the telescopic mechanism. When the detection cup 14 of the detection cup storage module 17 needs to be replaced, the telescopic mechanism is configured to move the detection cup storage module 17 out of the detection device, so as to facilitate the user to replace the detection cup 14. In this way, it may be possible to ensure that the detection device may replace the detection cup 14 without stopping the machine and maintain the continuity of the detection.

In some embodiments, the detection cup storage module 17 includes an automatic detection cup sorting mechanism. The automatic detection cup sorting mechanism is configured to automatically sort the detection cup, and move a sorted detection cup 14 to the grabbing module 18, such that detection cups 14 which are scattered may be placed in order. In addition, when the number of the detection cups 14 in the detection cup storage module 17 is insufficient, the detection cup storage module 17 may generate an alarm signal to remind the user to add a new detection cup 14 to the detection cup storage module 17.

In some embodiments, the first incubation detection channel 131 is an optical detection channel. As shown in FIG. 3, the detection cup 14 is arranged on the first incubation detection channel 131 to enable the optical detection module 30 to detect the sample and the single-serving detection reagent which are in the detection cup 14. The optical detection module 30 includes a light source generator 31 and a photodetector 32. The light source generator 31 is configured to emit a light beam, and the light beam irradiates the sample to be detected in the detection cup 14 to generate scattered light or transmitted light. The photodetector 32 is configured to detect the scattered light or the transmitted light, convert an optical signal of the scattered light or an optical signal of the transmitted light into an electrical signal, and the detection result is obtained. Therefore, the detection cup 14 applicable to the optical detection module 30 needs to be made of a material with good light transmittance. A cross-sectional shape of the detection cup 14 may be circular or rectangular, such that the detection cup 14 may be arranged on the first incubation detection channel 131.

In some embodiments, the first incubation detection channel 131 is a magnetic bead detection channel. As shown in FIG. 4, the detection cup 14 is arranged on the first incubation detection channel 131, such that the sample in the detection cup 14 and the single-serving detection reagent may be detected by the magnetic bead detection module 40. The magnetic bead detection module 40 includes an alternating coil 41 and a detection coil 42. A groove is defined on an inner wall of the detection cup 14, such that a magnetic bead may roll or oscillate in the groove. The alternating coil 41 is configured to drive the magnetic bead to roll. As the sample to be detected is coagulated, a oscillating amplitude of the magnetic bead becomes smaller and smaller. The detection coil 42 is arranged in a plane perpendicular to the alternating coil 41. The detection coil 42 is configured to detect an induced current generated by cutting the alternating coil 41 during a rolling process of the magnetic bead, and the detection result is obtained. The cross-sectional shape of the detection cup 14 may be circular or rectangular, such that the detection cup 14 may be arranged on the first incubation detection channel 131.

The optical detection module 30 is prone to be affected when interferents of chyle, jaundice, hemolysis, etc., occur in the sample to be detected, which leads to deviation of the detection result. The magnetic bead detection module 40 may not be affected by the substance in the sample to be detected, such as chyle, jaundice, hemolysis, etc.

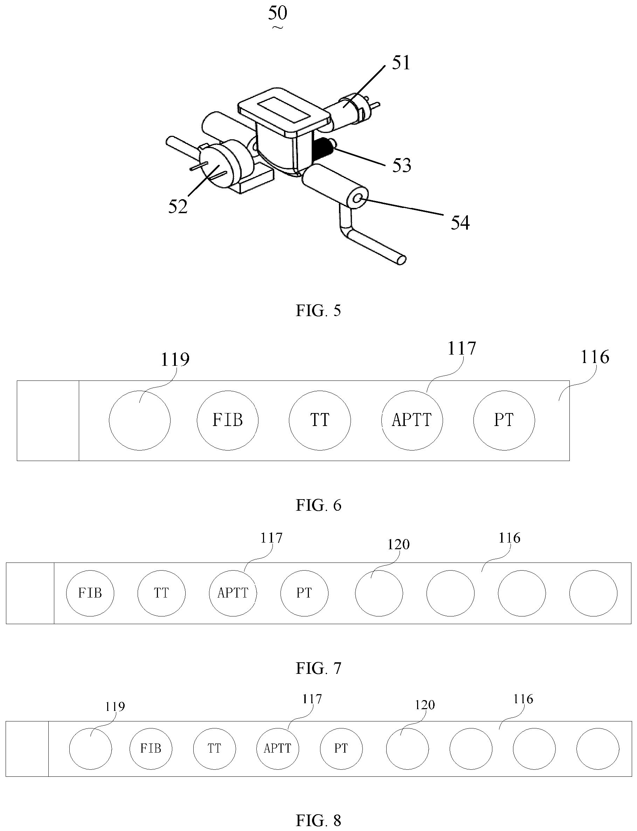

In some embodiments, the first incubation detection channel 131 is a combined detection channel for optical detection and magnetic bead detection. As shown in FIG. 5, the detection cup 14 is arranged on the first incubation detection channel 131, such that the sample in the detection cup 14 and the single-serving detection reagent may be detected by a combined detection module for optical detection and magnetic bead detection 50. The combined detection module for optical detection and magnetic bead detection 50 includes a light source generator 51, a photodetector 52, an alternating coil 53, and a detection coil 54. The light source generator 51 and the photodetector 52 are arranged opposite to each other, and are disposed above the alternating coil 53 and the detection coil 54. The detection coil 54 is arranged in a plane perpendicular to the alternating coil 53. Therefore, in the embodiments, an optical detection method and a magnetic bead detection method are combined in a same channel through the combined detection module for optical detection and magnetic bead detection 50, so as to reduce the number of channels in the first incubation detection channel 131.

In some embodiments, the detection device includes a display screen 19 configured to display an operation interface. In some embodiments, the operation interface displays a detection item that can be detected by the detection device, allowing the user to select the detect item on the operation interface. Alternatively, the operation interface is configured to display the detection result.

In some embodiments, the detection device includes a barcode scanner 20 configured to scan identification information of the reagent kit 116.

In some embodiments, the detection device includes a cleaning solution module 21, a waste liquid module 22, a power source 23, a cleaning pool 24, a printer 25, and a waste cup barrel 26. The power source 23 is configured to supply power to the detection device. The cleaning solution module 21 is configured to supply the cleaning solution for the detection device, and includes a cleaning solution barrel 211 and a first pump body 212. The first pump body 212 is configured to aspirate the cleaning solution from the cleaning solution barrel 211. The waste liquid module 22 is configured to collect the waste liquid of the detection device, and includes a waste liquid barrel 221 and a second pump body 222. The second pump body 222 is configured to aspirate the waste liquid of the detection device to the waste liquid barrel 221. The cleaning pool 24 is configured to clean the dispensing needle of the dispensing module 12. The printer 25 is configured to print the coagulation detection result. The waste cup barrel 26 is configured to place the detection cup 14 that has completed the detection.

The scanning module 16, the sample storage module 15, the reagent storage module 11, and the display screen 19 are sequentially arranged along a first direction of the detection device. The reagent storage module 11 and the incubation and detection module 13 are sequentially arranged along a second direction of the detection device. The first direction is substantially perpendicular to the second direction, such that a layout of the detection device is compact and a volume of the detection device is reduced.

In some embodiments, the detection device includes a needle storage area. A needle of the dispensing module 12 is a disposable tip. A dispensing arm of the dispensing module 12 retrieves a needle from the needle storage area. The dispensing arm of the dispensing module 12 may be a pneumatic apparatus, without the requirement for designing a fluidic system, such that costs may be reduced.

In an embodiment of the present disclosure, as shown in FIG. 6, the reagent kit 116 includes a third reagent kit, and the third reagent kit includes at least one reagent storage unit 117 and a sample storage unit 119. The at least one reagent storage unit 117 is configured to store the single-serving detection reagent, and the sample storage unit 119 is configured to store the sample, such as a single sample.

The at least one reagent kit storage channel 115 of the reagent storage module 11 includes a third reagent kit storage channel, and the third reagent kit storage channel is configured to store the third reagent kit.

As shown in FIG. 1, the first incubation detection channel 131 further corresponds to the third reagent kit storage channel. When the third reagent kit is stored in the third reagent kit storage channel to perform the single-serving coagulation detection, or when the detection device performs the multi-serving coagulation detection, the dispensing module 12 injects the sample and the single-serving detection reagent into the detection cup 14, respectively, or the dispensing module 12 injects the sample and the detection reagent in the reagent bottle 113 into the detection cup 14, respectively. The detection cup 14 is transferred to the first incubation detection channel 131. In some embodiments, the grabbing module 18 may be configured to transfer the detection cup 14 to the first incubation detection channel 131.

A detection process of the detection device for performing the single-serving coagulation detection based on the third reagent kit may be described as follows. The grabbing module 18 moves the detection cup 14 from the detection cup storage module 17 to the liquid dispensing position. The liquid dispensing position may be located in the detection cup storage module 17 or the incubation and detection module 13. The dispensing module 12 injects the sample in the sample storage unit 119 of the third reagent kit into the detection cup 14, and the dispensing module 12 is cleaned. The dispensing module 12 aspirates the single-serving detection reagent from the reagent storage unit 117 of the third reagent kit, and injects the single-serving detection reagent into the detection cup 14. The grabbing module 18 moves the detection cup 14 from the liquid dispensing position to the first incubation detection channel 131 of the incubation and detection module 13. After the sample in the detection cup 14 and the single-serving detection reagent is incubated, the sample in the detection cup 14 may be detected through the first incubation detection channel 131, so as to calculate the coagulation detection result such as coagulation time of the sample, a concentration of the sample, an activity indicator of the sample, etc. In addition, other components of the detection device may be the same as those of the detection device in the above-mentioned embodiments, which will not be repeated herein.

In an embodiment of the present disclosure, as shown in FIG. 7, the reagent kit 116 includes a second reagent kit, and the second reagent kit includes at least one reagent storage unit 117 and at least one detection unit/detection unit 120. The at least one reagent storage unit 117 is configured to store the single-serving detection reagent, and the at least one detection unit 120 is configured to serve as the detection cup 14. The dispensing module 12 injects the sample into the at least one detection unit 120, and injects the detection reagent in the reagent bottle 113 or the single-serving detection reagent in the second reagent kit into the at least one detection unit 120.

The at least one reagent kit storage channel 115 of the reagent storage module 11 includes a second reagent kit storage channel, and the second reagent kit storage channel is configured to store the second reagent kit.

As shown in FIG. 1, the incubation and detection module 13 includes a second incubation detection channel 132, and the second incubation detection channel 132 is arranged correspondingly to the second reagent kit storage channel. When the second reagent kit is placed into the storage channel of the second reagent kit, the at least one detection unit 120 in the second reagent kit is placed into the second incubation detection channel 132, such that the at least one detection unit 120 is configured to serve as the detection cup 14, and the dispensing module 12 injects the sample and the single-serving detection reagent into the at least one detection unit 120, respectively, or the dispensing module 12 injects the sample and the detection reagent in the reagent bottle 113 into the at least one detection unit 120, respectively.

A detection process of the detection device for performing the single-serving coagulation detection based on the second reagent kit may be described as follows. As shown in FIGS. 1 and 7, the dispensing module 12 injects the sample in the test tube 151 of the sample storage module 15 into a corresponding one of the at least one detection unit 120, and the dispensing module 12 is cleaned. The dispensing module 12 aspirates the single-serving detection reagent from the reagent storage unit 117 of the second reagent kit, and injects the single-serving detection reagent into the corresponding one of the at least one detection unit 120. After the sample in the corresponding one of the at least one detection unit 120 and the single-serving detection reagent is incubated, the sample in the corresponding one of the at least one detection unit 120 may be detected through the second incubation detection channel 132, so as to calculate the coagulation detection result such as coagulation time of the sample, a concentration of the sample, an activity indicator of the sample, etc.

In some embodiments, the second incubation detection channel 132 is the optical detection channel, the magnetic bead detection channel, or the combined detection channel for optical detection and magnetic bead detection. The second incubation detection channel 132 is the same as the first incubation detection channel 131, which will not be repeated herein.

In an embodiment of the present disclosure, as shown in FIG. 8, the reagent kit 116 includes a fourth reagent kit, the fourth reagent kit includes the at least one reagent storage unit 117, the sample storage unit 119, and the at least one detection unit 120. The at least one reagent storage unit 117 is configured to store the single-serving detection reagent, the sample storage unit 119 is configured to store the sample, and the detection unit 120 is configured to serve as the detection cup 14. The dispensing module 12 injects the sample into the at least one detection unit 120, and injects the detection reagent in the reagent bottle 113 or the single-serving detection reagent in the fourth reagent kit into the at least one detection unit 120.

The at least one reagent kit storage channel 115 of the reagent storage module 11 includes a fourth reagent kit storage channel, and the fourth reagent kit storage channel is configured to store the fourth reagent kit.

As shown in FIG. 1, the second incubation detection channel 132 is arranged correspondingly to the fourth reagent kit storage channel. When the fourth reagent kit is placed into the storage channel of the fourth reagent kit, the at least one detection unit 120 in the fourth reagent kit is placed into the second incubation detection channel 132, such that the at least one detection unit 120 is configured to serve as the detection cup 14, and the dispensing module 12 injects the sample and the single-serving detection reagent into the detection unit 120, respectively, or the dispensing module 12 injects the sample and the detection reagent in the reagent bottle 113 into the detection unit 120, respectively.

A detection process of the detection device for performing the single-serving coagulation detection based on the fourth reagent kit may be described as follows. As shown in FIGS. 1 and 8, the dispensing module 12 injects the sample in the sample storage unit 119 of the fourth reagent kit into a corresponding one of the at least one detection unit 120, and the dispensing module 12 is cleaned. The dispensing module 12 aspirates a single-serving detection reagent from the reagent storage unit 117 of the fourth reagent kit, and injects the single-serving detection reagent into the corresponding one of the at least one detection unit 120. After the sample in the corresponding one of the at least one detection unit 120 and the single-serving detection reagent is incubated, the sample in the corresponding one of the at least one detection unit 120 may be detected through the second incubation detection channel 132, so as to calculate the coagulation detection result such as coagulation time of the sample, a concentration of the sample, an activity indicator of the sample, etc.

A reagent kit may be further provided by the embodiments of the present disclosure. As shown in FIG. 2, the reagent kit 116 includes a main body, the at least one reagent storage unit 117, and a handle 118. The at least one reagent storage unit 117 is arranged on the main body. The handle 118 is arranged at an end of the main body. The at least one reagent storage unit 117 is configured to store the single-serving detection reagent, such that the single-serving coagulation detection may be performed by the detection device, which is the same as the above-mentioned embodiments, and which will not be repeated herein. At this time, the reagent kit 116 is configured to serves as the first reagent kit of the above-mentioned embodiments.

In some embodiments, as shown in FIG. 6, the reagent kit 116 includes the at least one reagent storage unit 117 and the sample storage unit 119. Each of the at least one reagent storage unit 117 is configured to store the single-serving detection reagent. The sample storage unit 119 is configured to store the sample, such as the single sample. At this time, the reagent kit 116 is configured to serves as the third reagent kit of the above-mentioned embodiments.

In some embodiments, as shown in FIG. 7, the reagent kit 116 includes the at least one reagent storage unit 117 and the at least one detection unit 120. Each of the at least one reagent storage unit 117 is configured to store the single-serving detection reagent. Each of the at least one detection unit 120 is configured to serve as the detection cup 14. At this time, the reagent kit 116 is configured to serves as the second reagent kit of the above-mentioned embodiments.

In some embodiments, as shown in FIG. 8, the reagent kit 116 includes the at least one reagent storage unit 117, the sample storage unit 119, and the at least one detection unit 120. Each of the at least one reagent storage unit 117 is configured to store the single-serving detection reagent. The sample storage unit 119 is configured to store the sample. Each of the at least one detection unit 120 is configured to serve as the detection cup 14. At this time, the reagent kit 116 is configured to serves as the fourth reagent kit of the above-mentioned embodiments.

In conclusion, the reagent storage module 11 provided in the embodiments of the present disclosure includes the multi-serving reagent storage module 111 and the single-serving reagent storage module 112. The multi-serving reagent storage module 111 is configured to store the reagent bottle 113, so as to perform the multi-serving coagulation detection. The single-serving reagent storage module 112 includes the at least one reagent kit storage channel 115, and the at least one reagent kit storage channel 115 is configured to store the reagent kit 116. The reagent kit 116 includes the at least one reagent storage unit 117, and the at least one reagent storage unit 117 is configured to store the single-serving detection reagent, so as to perform the single-serving coagulation detection. Therefore, the detection device may store the reagent kit 116 through the single-serving reagent storage module 112, and the at least one reagent storage unit 117 of the reagent kit 116 is configured to store the single-serving detection reagent, such that the single-serving detection reagent may be supplied to the detection device without using the reagent bottle 113 in the multi-serving reagent storage module 111, thereby enabling the detection device to achieve a single sample detection, and thus it may be further possible to reduce waste of a large bottle of reagent. The detection device may improve the diversity of a detection method thereof, such that it may be possible to meet detection requirements of a user in different situations, thereby improving the user experience and the convenience of detection.

The above are only some embodiments of the present disclosure, not to limit the scope of the present disclosure. Any equivalent structure or equivalent process transformation made by using the contents of the specification and the accompanying drawings of the present disclosure, or directly or indirectly applied in other related technical fields, are included in the scope of the present disclosure in the same way.

## Claims

1. A detection device for detecting a coagulation item, comprising:
a reagent storage module, comprising a single-serving reagent storage module, wherein the single-serving reagent storage module comprises at least one reagent kit storage channel to store a reagent kit, and the reagent kit comprises at least one reagent storage unit to store a single-serving detection reagent, so as to perform a single-serving coagulation detection;
a dispensing module, configured to inject a collected sample into a detection cup, and inject the single-serving detection reagent in the reagent kit into the detection cup; and
an incubation and detection module, configured to incubate the sample in the detection cup, and detect the sample in the detection cup to obtain a coagulation detection result.

2. The detection device according to claim 1, further comprising:
a sample storage module, configured to store a test tube, wherein the dispensing module is configured to inject a sample in the test tube into the detection cup; and
a scanning module, configured to scan identification information of the test tube, so as to enable the detection device to bind the identification information with the coagulation detection result.

3. The detection device according to claim 1 or 2, further comprising a detection cup storage module configured to store the detection cup, wherein the dispensing module is configured to inject the collected sample into the detection cup in the detection cup storage module, and inject the single-serving detection reagent in the reagent kit into the detection cup in the detection cup storage module.

4. The detection device according to claim 3, further comprising a grabbing module configured to move the detection cup from the detection cup storage module to the incubation and detection module.

5. The detection device according to any one of claims 1-4, wherein the reagent storage module comprises a multi-serving reagent storage module, the multi-serving reagent storage module is configured to store a reagent bottle to perform a multi-serving coagulation detection, and the dispensing module is configured to inject the detection reagent in the reagent bottle or the single-serving detection reagent in the reagent kit into the detection cup.

6. The detection device according to any one of claims 1-5, wherein the reagent kit comprises a first reagent kit, and the first reagent kit comprises at least one reagent storage unit configured to store the single-serving detection reagent; and the at least one reagent kit storage channel comprises a first reagent kit storage channel configured to place the first reagent kit.

7. The detection device according to any one of claims 1-6, wherein the reagent kit comprises a second reagent kit, the second reagent kit comprises at least one reagent storage unit and at least one detection unit, the at least one reagent storage unit is configured to store the single-serving detection reagent, and the at least one detection unit is configured to serve as the detection cup;
the dispensing module is configured to inject the sample into the detection unit, and inject the detection reagent in the reagent bottle or the single-serving detection reagent in the reagent kit into the detection unit; and
the at least one reagent kit storage channel comprises a second reagent kit storage channel configured to place the second reagent kit.

8. The detection device according to any one of claims 1-7, wherein the reagent kit comprises a third reagent kit, the third reagent kit comprises at least one reagent storage unit and a sample storage unit, the reagent storage unit is configured to store the single-serving detection reagent, and the at least one sample storage unit is configured to store the sample; and
the at least one reagent kit storage channel comprises a third reagent kit storage channel configured to place the third reagent kit.

9. The detection device according to any one of claims 1-8, wherein the reagent kit comprises a fourth reagent kit, the fourth reagent kit comprises at least one reagent storage unit, at least one detection unit, and a sample storage unit, the reagent storage unit is configured to store the single-serving detection reagent, the sample storage unit is configured to store the sample, and the detection unit is configured to serve as the detection cup;
the dispensing module is configured to inject the sample into the detection unit, and inject the detection reagent in the reagent bottle or the single-serving detection reagent in the reagent kit into the detection unit; and
the at least one reagent kit storage channel comprises a fourth reagent kit storage channel configured to place the fourth reagent kit.

10. The detection device according to any one of claims 6-9, wherein the incubation and detection module comprises a first incubation detection channel, and in response to the first reagent kit being stored in the first reagent kit storage channel or the third reagent kit being stored in the third reagent kit storage channel to perform the single-serving coagulation detection, or in response to the detection device performing the multi-serving coagulation detection, the dispensing module is configured to inject the sample and the single-serving detection reagent into the detection cup, respectively, or the dispensing module is configured to inject the sample and a detection reagent in the reagent bottle into the detection cup, respectively, and the detection cup is transferred to the first incubation detection channel.

11. The detection device according to any one of claims 6-9, wherein the incubation and detection module comprises a second incubation detection channel arranged correspondingly to the second reagent kit storage channel and/or the fourth reagent kit storage channel, in response to the second reagent kit being placed into the second reagent kit storage channel or the fourth reagent kit being placed into the fourth reagent kit storage channel, the at least one detection unit in the second reagent kit or the at least one detection unit in the fourth reagent kit is placed into the second incubation detection channel, such that the at least one detection unit in the second reagent kit or the at least one detection unit in the fourth reagent kit is configured to serve as the detection cup, and the dispensing module is configured to inject the sample and the single-serving detection reagent into the at least one detection unit in the second reagent kit or the at least one detection unit in the fourth reagent kit, or the dispensing module is configured to inject the sample and the detection reagent in the reagent bottle into the at least one detection unit in the second reagent kit or the at least one detection unit in the fourth reagent kit.

12. The detection device according to claim 10, wherein the first incubation detection channel is an optical detection channel.

13. The detection device according to claim 12, wherein the optical detection channel comprises an optical detection module; and
the optical detection module comprises:
a light source generator, configured to emit a light beam, wherein the light beam irradiates to the sample to be detected in the detection cup to generate scattered light or transmitted light; and
a photodetector, configured to detect the scattered light or the transmitted light, and configured to convert an optical signal of the scattered light or an optical signal of the transmitted light into an electrical signal to obtain the detection result.

14. The detection device according to claim 10, wherein the first incubation detection channel is a magnetic bead detection channel.

15. The detection device according to claim 14, wherein the magnetic bead detection channel comprises a magnetic bead detection module, and a groove is defined on the detection cup, so as to cause a magnetic bead to roll in the groove; and
the magnetic bead detection module comprises:
an alternating coil, configured to drive the magnetic bead to roll; and
a detection coil, arranged in a plane perpendicular to the alternating coil, and configured to detect an induced current which is generated by cutting the alternating coil during a rolling process of the magnetic bead to obtain the detection result.

16. The detection device according to claim 10, wherein the first incubation detection channel is a combined detection channel for optical detection and magnetic bead detection.

17. The detection device according to claim 16, wherein the combined detection channel for optical detection and magnetic bead detection comprises: a light source generator, a photodetector, an alternating coil, and a detection coil, the light source generator and the photodetector are arranged opposite to each other and are disposed above the alternating coil and the detection coil, and the detection coil is arranged in a plane perpendicular to the alternating coil.

18. A reagent kit, applicable to the detection device according to any one of claims 1-17, and the reagent kit comprising a main body and the at least one reagent storage unit arranged on the main body, wherein the at least one reagent storage unit is configured to store the single-serving detection reagent, such that the detection device is configured to perform the single-serving coagulation detection.

19. The reagent kit according to claim 18, wherein the reagent kit further comprises a handle arranged at an end of the main body.

20. The reagent kit according to claim 18, wherein the reagent kit comprises a sample storage unit configured to place the sample.

21. The reagent kit according to any one of claims 18-20, wherein the reagent kit comprises at least one detection unit configured to serve as the detection cup of the detection device.
